# NEUE EUROPÄISCHE PATENTSCHRIFT

(11) **EP 0 816 345 B2**
(45) Veröffentlichungstag und Bekanntmachung des Hinweises auf die Entscheidung über den Einspruch: **18.01.2006**
(45) Hinweis auf die Patenterteilung: 29.01.2003
(21) Anmeldenummer: 97107402.6
(22) Anmeldetag: 05.05.1997
(51) Int. Cl.: C07D 239/54

(54) **Verfahren zur Herstellung von 4,6-Dihydroxypyrimidin**
Process of preparation of 4,6-Dihydroxypyrimidine
Procédé pour la préparation de 4,6-Dihydroxypyrimidine

(30) Priorität: 03.07.1996 DE 19626747; 02.10.1996 DE 19640756
(43) Veröffentlichungstag der Anmeldung: 07.01.1998
(73) Patentinhaber: Degussa AG, 40474 Düsseldorf (DE)
(72) Erfinder: Hunds, Artur, Dr., 53121 Bonn (DE)

(56) Entgegenhaltungen:
- WO-A-97/44327
- CN-A- 1 082 031
- DE-B- 1 200 308
- GB-A- 1 092 144
- B. A. ZASONOV ET AL.: "Synthesis of 4-(n-aminobenzolsulphamido)-6-methoxypyrim idine(Russian)" KHIM. FARM. ZH., Bd. 8, Nr. 12, 1974, MOSKAU, Seiten 28-31, XP002045819
- D. J. BROWN: "Pyrimidine Reactions. Part I. Pyrimidines from Malondiamide" JOURNAL OF CHEMICAL SOCIETY, 1956, LONDON, Seiten 2312-2314, XP002020558
- CLAUDE HENNART ET AL.: "Contribution à la synthèse de la dichloro-4.6-pyrimidine" BULLETIN DE LA SOCIET¹ CHIMIQUE DE FRANCE, 1959, PARIS, Seiten 741-742, XP000611480
- R. HULL: "A new Synthesis of 4:6-Dihydroxypyrimidines" JOURNAL OF CHEMICAL SOCIETY, 1951, Seite 2214 XP002019800

## Beschreibung

Gegenstand der Erfindung ist ein verbessertes Verfahren zur Herstellung von 4,6-Dihydroxypyrimidin (DHP), in seiner tautomeren Form auch 1-H-Pyrimidin-4,6-dion genannt, aus Malonsäureestern, Formamid und Alkoholäten. DHP ist ein wertvolles Zwischenprodukt für Wirkstoffsynthesen. So kann man aus 4.6-Dihydroxypyrimidin das entsprechende Dichlorpyrimidin herstellen, das seinerseits zu neuen, hochwinksamen Fungiziden verarbeitet werden kann (EP-A1 Nrn. 0 382 375. 0 393 861. 0468 684 und 0 468 695).

Bei dem bekannten Verfahren zur Herstellung von DHP nach R. Hull. J.Chem.Soc., 1951. 2214, werden Malonsäurediamid und eine Lösung von Natriumethanolat in Ethanol bei Raumtemperatur zusammengegeben. Ethylformiat wird zugesetzt, und dann wird das Gemisch 2 Stunden zum Rückfluß erhitzt. Natriumethanolat und Ethylformiat werden im Mengen von 1.5 bzw. 2 Mol je Mol Malonsäurediamid eingesetzt. Die ausgefallenen Salze werden abgetrennt und in Wasser gelöst, und aus der Lösung wird DHP durch Zusatz von Säure ausgefällt und abgetrennt. Die Ausbeute an DHP beträgt ca. 40 % d.Th.. Diese Ergebnisse wurden durch C. Hennart et al., Bull.Soc.Chim., 1959. 741, bestätigt, die nur 1 mol Ethylformiat einsetzten und eine Rohausbeute an DHP von 44 % d.Th. erzielten.

D.J. Brown. J.Chem.Soc., 1956 hat die Synthese von Hull modifiziert, indem er das Ethylformiat durch 1,6 Mol Formamid ersetzte, wodurch die Ausbeute auf ca. 52 % d.Th. gesteigert wurde.

Eine Verbesserung der Ausbeute an DHP um ca. 30 % auf maximal 81% erzielte A. Sömmer, DE-OS 1 200 308, dadurch, daß er Malonsäurediamid mit Formamid in alkoholischer Lösung mit mehr als 2 Mol. vorteilhaft mit 3.0 bis 3.9 Mol Alkalialkoholat umsetzte. Dabei wurde in den Beispielen, anders als bei Brown, Formamid in Mengen von etwas mehrals 2 Mol je Mol Malonsäurediamid angewandt. Als Alkalialkoholate wurden Natriummethanolat in Methanol und Natriumethanolat in Ethanol verwendet. Im letzteren Falle wurde das Methanol aus dem Reaktionsgemisch abdestilliert, wodurch die Filtration der ausgefallenen Salze entfiel. Ameisensäureethylester an Stelle von Formamid lieferte mit Natriumethanolat DHP in einer Ausbeute von nur 61,5 %.

Die Herstellung von DH P nach A.Sömmer, loc cit., läßt sich durch das folgende summarische Formelschema beschreiben:

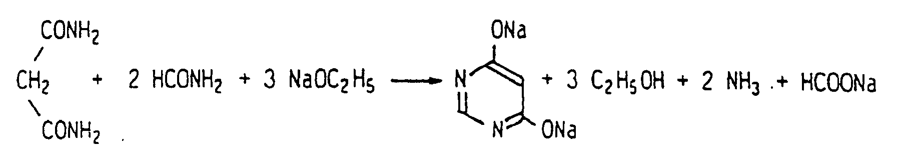

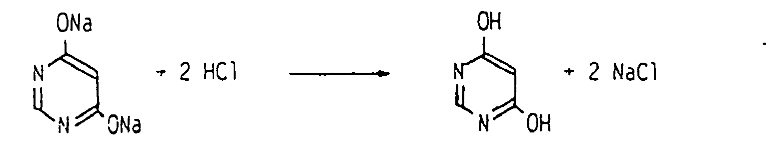

Ähnliche Ergebnisse werden von V.A.Zasonov et al. in Khim.-Farm. Zh. Vol. 8. Nr. 12. 28-31 berichtet. Mit 2 Mol Formamid und 3 Mol Natriumethylatje Mol Malonsäurediamid erzielten sie eine DHP-Ausbeute von 83.2 %. Zasosov et al. führen auch ein Beispiel für die Herstellung des Ausgangsstoffs Malonsäurediamid mit einer Ausbeute von 95 % aus Malonsäureester und Ammoniak auf. Das Malonsäurediamid wird dabei vor seiner Umsetzung zu DHP isoliert. Es handelt sich also um eine Aggregalion zweier Verfahrensstufen, die in keiner Weise integriert sind. Die DHP-Ausbeute, bezogen auf Malonsaureester, beträgt 79 % d.Th.

Nachteilig bei allen bisher genannten Verfahren ist der Umstand, daß der Ausgangsstoff Malonsäurediamid nicht in kommerziellen Mengen verfügbar ist. Bei den Verfahren von Hull. Hennart et al. und Brown sind die Ausbeuten unbefriedigend. Die Verfahren von A.Sömmer und von V.A.Zasosov et al. liefern mit Formamid zwar bessere, wenn auch nicht voll befriedigende Ausbeuten, erfordern aber wie die zuvor erwähnten Verfahren als Ausgangsstoff Malonsäurediamid, das aus Malonsäureester hergestellt wird. Dadurch und durch das Formamid werden also schon nach der Stöchiometrie 4 Mole Stickstoff eingebracht, wovon sich im günstigsten Fall (V.A.Zasosov et al., Ausbeute 83.2%) 1.664 Mole im DHP wiederfinden. Mehr als die Hälfte des Stickstoffs geht also in Form von Ammoniak in Methanol, Ethanol oder das Abwasser. Mit Ammoniak verunreinigtes Methanol bzw. Ethanol wiederverwendbar aufzuarbeiten ist schwierig und aufwendig. Weiterhin ist die Abtrennung des DHP-Na-Salzes durch Filtration problematisch, weil die Salze oft sehr feinkristallin und daher schwierig filtrierbar anfallen. Schließlich ist die DHP-Konzentration im Reaktionsgemisch und damit die Raum-Zeit-Ausbeute niedrig. Aus 100 Gewichtsteilen Reaktionsgemisch erhält man bestenfalls ca. 7 Gewichtsteile DHP.

Ein verbessertes Verfahren zur Herstellung von DHP wird von Kyowa Hakko in GB-A2-1 092144 beschrieben. Dabei geht man von einem Malonsäurealkylester aus, der in Gegenwart eines Alkalikatalysators mit Ammoniak oder Formamid zum Malonsäurediamid umgesetzt wird, das dann mit Formamid zu DHP kondensiert wird. Es handelt sich also um ein zweistufiges Verfahren, das jedoch, anders als bei Zasosov et al., in demselben Reaktor ohne Isolierung des Produkts der ersten Stufe durchgeführt wird. Als Alkalikatalysatoren werden Kalium- und Natriumethanolat verwendet, und es sollen pro Mol Malonsäureester insgesamt nicht wesentlich weniger als 3 Mol Ammoniak oder Formamid eingesetzt werden. Die Ausbeuten in den 4 Beispielen errechnen sich zu 75 bis 90% d.Th.. Allerdings ergab eine Nacharbeitung des Beispiels mit der besten Ausbeute, daß die Natriumsalze sehr schlecht filtrierbar waren und die Ausbeute an DHP tatsächlich nur 85% d.Th. beträgt.

Obwohl das Kyowa Hakko-Verfahren gute Ausbeuten liefert und von den in kommerziellen Mengen erhältlichen Malonsäureestern ausgeht, ist es dennoch insgesamt nicht voll befriedigend. Wie die Beispiele zeigen, ist die Herstellung des Malonsäurediamids in der ersten Stufe problematisch Entweder sind, wie in den Beispielen 1 und 2. Reaktioriszelten von 48 Stunden erforderlich, was die Raum-Zeit-Ausbeute herabsetzt. Oder es gibt Rührprobleme, wie im Beispiel 3 beschrieben. Im Beispiel 4 wird mit festem Kaliumethanolat gearbeitet, das hygroskopisch und daher schlecht zu handhaben ist nicht selten ätzende Stäube bildet und selbstentzündlich sein kann. Weiterhin sind zur Erzielung hoher Ausbeuten erheblich mehr als die im ersten Patentanspruch geforderten 3 Mol Ammoniak oder Formamid je Mol Malonsäurealkylester erforderlich. Im Beispiel 3 werden 7,7, im Beispiel 4 sogar 10.1 Mol Formamid je Mol Malonsäurediethylester eingesetzt. Daher erhält man nach dem Abfiltrieren der Salze stark mit Ammoniak und Formamid belastete Filtrate. Auch ist, wie bei den zuvor beschriebenen Verfahren, die Konzentration des DHP im Reaktionsgemisch niedrig, sie beträgt im besten Fall (Beispiel 3) 6 g DHP auf 100g Reaktionsgemisch. Entsprechend gering sind die Raum-Zeit-Ausbeuten.

Es wurde nun gefunden, daß sich 46-Dihydroxypyrimidin durch Umsetzung eines Malonsäureesters mit Formamid und einem Alkalimetallalkoholat bei erhöhter Temperatur vorteilhaft herstellen läßt, wenn der Malonsäureester allein oder gleichzeitig mit der Gesamtmenge oder einer Teilmenge des Formamids anteilsweise oder kontinuierlich dem als Lösung oder Suspension in einem Alkohol allein oder zusammen mit der Gesamtmenge oder der restlichen Teilmenge des Formamids vorgelegten Alkalimetallalkoholat zugefügt wird und dass die Reaktion unter dem Druck durch geführt wird, der sich bei der betreffenden Reaktionstemperatur einstellt.

Für den Fall der Umsetzung des Malonsäuredimethylesters mit Formamid und Natriummethanolat läßt sich die Reaktionsgleichung summarisch wie folgt formulieren:

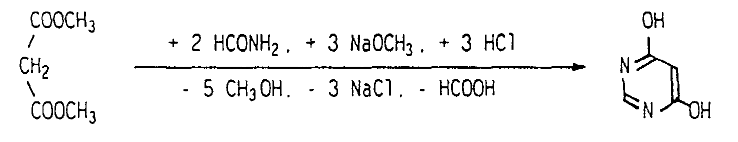

Das Verfahren nach der Erfindung weist eine Reihe von überraschenden Vorteilen gegenüber den Verfahren des Standes der Technik auf. Es geht statt von Malonsäurediamid von den in kommerziellen Mengen verfügbaren Malonsäureestern aus und führt in einem integrierten Verfahren in nur einer Reaktionsstufe in sehr guten Ausbeuten zum Alkalisalz des gewünschten DHP, das zur Freisetzung des DHP mittels Säure nicht aus dem Reaktionsgemisch abgetrennt zu werden braucht. Das Verfahren verbraucht für den Ringschluß zum Pyrimidinring weniger Amin- bzw. Amidstickstoff als die bekannten Verfahren. Man kann ohne aufwendige Maßnahmen Ammoniak-freien Alkohol zurückgewinnen, und die Belastung des Abwassers mit Ammoniak oder Ammoniumverbindungen wird gering gehalten. Die Konzentration des DHP im Reaktionsgemisch und damit die Raum-Zeit-Ausbeute ist deutlich höher als bei den bekannten Verfahren.

Die als Ausgangsstoffe verwendeten Malonsäureester sind bekannte, wohlfeile Stoffe und in kommerziellen Mengen verfügbar. Ein besonders bevorzugter Ausgangsstoff ist Malonsäuredimethylester.

Formamid kann als hochreines oder als technisch reines Produkt, wie es bei der technischen Synthese aus Methylformiat und Ammoniak erhalten wird, eingesetzt werden. Das Formamid wird zweckmäßig in Mengen von 2,0 bis 2,5, insbesondere von 2,1 bis 2,25 Mol je Mol Malonsäureester verwendet. Höhere Formamidmengen sind ohne weiteres möglich, widersprechen jedoch dem Ziel, die Verunreinigung der anfallenden Flüssigkeiten mit stickstoffhaltigen Stoffen gering zu halten. Niedrigere Formamidmengen sind möglich, doch geht dann die Ausbeute an DHP zurück, da zum Pyrimidinringschluß 2 Mol Stickstoffverbindung erforderlich sind.

Bevorzugte Alkalimetallalkoholate sind die Kalium- und insbesondere die Natriumälkoholate, die sich von Alkanolen mit 1 bis 4 Kohlenstoffatomen ableiten. Vorteilhaft werden sie als Lösungen oder Suspensionen in demjenigen Alkohol angewandt, von dem sie sich ableiten. Alkanole mit 1 bis 4 Kohlenstoffatomen sind dementsprechend die für das Verfahren bevorzugten Alkohole. Besonders bevorzugt wird Natriummethanolat, das als preisgünstige, kommerziell verfügbare Lösung von etwa 30 Gewichtsprozent in Methanol verwendet werden kann. Es können aber auch höherprozentige Lösungen oder Suspensionen von Natriumoder Kaliummethanolat eingesetzt werden, ohne daß Rührprobleme oder unkontrollierbare Verfestigungen des Reaktionsgemisches auftreten. Auch ist die Verwendung anderer Alkoholate. z.B. der bei den bekannten Herstellverfahren verwendeten Natrium- und Kallumethanolate, ohne weiteres möglich, wird aber wegen des höheren Preises nicht bevorzugt. Die Menge des mit dem Alkalialkoholat vorgelegten Alkohols (bzw. die Konzentration des gelösten oder gelösten und suspendierten Alkalialkoholats) bestimmt den Gehalt des Reaktionsgemisches an DHP-Alkalisalz und damit die Raum-Zeit-Ausbeute. Es ist ein Vorteil des Verfahrens nach der Erfindung, daß hochprozentige Alkalialkoholat-Lösungen oder -Suspensionen eingesetzt werden können. So erhält man bei Verwendung von ca. 40 Gewichtsprozent Natriummethanolat in Methanol nach dem Ansäuern DHP in einer Ausbeute von 14,5 g/100 g Reaktionsgemisch.

Das Verfahren nach der Erfindung wird ausgeführt, indem man den Malonsäureester, allein oder gleichzeitig mit der gesamten oder einer Teilmenge des Formamids, anteilsweise oder kontinuierlich dem als Lösung oder Suspension in einem Alkohol vorgelegten Alkalialkoholat und gegebenenfalls der gesamten oder der restlichen Teilmenge des Formamids zufügt. Es ist ein wesentliches Merkmal der Erfindung, daß der Malonsäureester, entweder in kleinen Anteilen oder vorteilhaft kontinuierlich, dem vorgelegten Alkalialkoholat zugefügt wird. Dagegen ist es unerheblich, ob das Formamid mit dem Alkalialkoholat vorgelegt oder aber diesem zugefügt wird. Daher kann das Formamid gegebenenfalls in beliebigem Verhältnis auf Vorlage und Zulauf verteilt werden. Wenn man den Malonsäureester gleichzeitig mit der gesamten Menge oder einer Teilmenge des Formamids dem vorgelegten Alkalialkoholat zufügt, dann kann das Formamid getrennt von dem Malonsäureester oder vorteilhaft im Gemisch mit diesem zugefügt werden.

Die Temperatur im Reaktionsgemisch wird zweckmäßig im Bereich von 30 bis 100°C. insbesondere von 50 bis 80°C gehalten. Die Reaktion ist schwach exotherm, so daß man gegebenenfalls kühlen muß, sobald mit der Zugabe von Malonsäureester begonnen wurde. Je nach den eingesetzten Stoffmengen nimmt die Zugabe des Malonsäureesters und gegebenenfalls des Formamids im allgemeinen etwa 15 bis 60 Minuten in Anspruch. Vorteilhaft läßt man das Reaktionsgemisch nach beendeter Zugabe einige Zeit. z.B. 30 Minuten bis 2 Stunden, bei einer Temperatur am oberen Ende des genannten Bereiches. z.B. bis 90 bis 100°C. nachreagieren.

Während der Reaktion und der Nachreaktion arbeitet man bei dem Druck, der sich bei der jeweiligen Temperatur einstellt, d.h. im allgemeinen bei 1 bis 5 bar.

Das Reaktionsgemisch wird nach dem Abkühlen mit Wasser versetzt, und das freie DHP wird durch Zusatz einer Säure, zweckmäßig einer Mineratsäure, wie Schwefelsäure. Phosphorsäure und insbesondere Salzsäure, aus seinem Alkalisalz freigesetzt. Man kann auch, wie in der DE-OS 1 200 308 beschrieben, das alkoholische Reaktionsgemisch zur Trockne eindampfen, wozu in der Technik z.B. vorteilhaft Dünnschichtverdampfer verwendet werden, die zurückbleibenden Salze in Wasser lösen und dann das DHP wie oben durch Säurezusatz freisetzen. Das ausgefällte DHP wird in beiden Fällen mit Wasser gewaschen und zweckmäßig bei erhöhter Temperatur, wie 50 bis 90°C. und unter vermindertem Druck, wie 20 bis 200 mbar, getrocknet. Man erhält das Reaktionsprodukt mit Ausbeuten, die 90 % d.Th. überschreiten können.

Die folgenden Beispiele sollen die Erfindung weiter erläutern, jedoch nicht ihren Umfang begrenzen, wie er in den Patentansprüchen dargelegt ist.

### Beispiel 1

In einem 5-1 Drehrührautoklaven werden 8.25 Mol Natriummethanolat In Form einer 30 Gew.%-igen Lösung in Methanol sowie 5.25 Mol Formamid vorgelegt, und das Gemisch wird auf 50°C erwärmt. Dann werden Innerhalb von 60 Minuten 2.50 Mol Malonsäuredimethylester kontinuierlich zugepumpt, wobei die Temperatur auf 65°C steigt Man läßt eine Stunde bei 95°C nachreagieren, entspannt den Autoklaven und spült ihn mit Stickstoff. Danach wird das Reaktionsgemisch mit 1.150 ml Wasser versetzt, und 7.0 Mol wäßrige 36 Gew.%-ige Salzsäure werden zugetropft, wobei die Temperatur durch Kühlen auf 20 bis 25°C gehalten wird. Das ausgefällte DHP wird abgesaugt und dreimal mit Wasser gewaschen. Nach dem Trocknen bei 70-80°C/20-30 mbar erhält man 235,4g DHP. entsprechend einer Ausbeute von 84,0 % d.Th.. Von den 5,25 Mol Stickstoff, die als Formamid eingesetzt wurden, sind nur 1,05 Mol verlorengegangen, entsprechend 62,5 g pro kg DHP. Im bisher günstigsten, von V.A.Zasosov et al. beschriebenen Beispiel gingen pro kg DHP 351 g Stickstoff verloren.

### Beispiel 2

In einem 5-1-Drehrührautoklaven werden 8,25 Mol in Methanol gelöstem bzw. suspendiertem Natriummethanolat (Feststoffgehalt 41,4 Gew.%) vorgelegt und auf 75°C erwärmt. Dann wird innerhalb von 60 Minuten eine Mischung aus 2,5 Mol Malonsäuredimethylester und 5,6 Mol Formamid kontinuierlich zugepumpt, wobei die Temperatur auf 85°C ansteigt.
Man läßt wie im Beispiel 1 nachreagieren und arbeitet das Reaktionsgemisch durch Zusatz von 900 ml Wasser und 7,0 Mol wäßriger 36 Gew.%-iger Salzsäure in analoger Weise auf. Die Ausbeute beträgt 246,6 g, entsprechend 88,0 % d.Th.. Der Stickstoffverlust pro kg DHP beträgt 68,2 g.

### Beispiel 3

In einem 51-Drehrührautoklaven werden 8,25 Mole einer41,4 %-igen Suspension von Natriummethanolat in Methanol und 5,60 Mole Formamid vorgelegt und auf 60°C erwärmt. Dann werden innerhalb von 60 Minuten 2,50 Mole Malonsäuredimethylester zugepumpt, wobei die Temperatur langsam auf 65°C ansteigt. Man läßt das Reaktionsgemisch wie im Beispiel 1 nachreagieren und arbeitet es mit 900 ml Wasser und 7.0 Molen wäßriger 36%-iger Salzsäure in analoger Weise auf. Die Ausbeute beträgt 254,7 g. entsprechend 90.9 % d.Th.. Pro kg DHP sind 58 g Stickstoff verlorengegangen.

## Patentansprüche

1. Verfahren zur Herstellung von 4,6-Dihydroxypyrimidin durch Umsetzung Malonsäureesters mit Formamid und einem Alkalimetallalkoholat bei 30 bis 100 °C,
**dadurch gekennzeichnet,**
**dass** der Malonsäureester allein oder gleichzeitig mit der Gesamtmenge oder einer Teilmenge des Formamids portionsweise oder kontinuierlich dem als Lösung oder Suspension in einem Alkohol allein oder zusammen mit der Gesamtmenge oder der restlichen Teilmenge des Formamids vorgelegten Alkalimetallalkoholat zugefügt wird und dass die Reaktion unter dem Druck durchgeführt wird, der sich bei der betreffenden Reaktionstemperatur einstellt.

2. Verfahren nach Anspruch 1,
**dadurch gekennzeichnet,**
**dass** die Temperatur im Reaktionsgemisch 50 bis 80 °C beträgt, während der Malonsäureester zugefügt wird.

3. Verfahren nach Anspruch 1 oder 2,
**dadurch gekennzeichnet,**
**dass** pro Mol Malonsäureester 2,0 bis 2,5, vorzugsweise 2,1 bis 2,25 Mol Formamid und 3,0 bis 4,0, vorzugsweise 3,0 bis 3,5 Mol Alkalimetallalkoholat verwendet werden.

4. Verfahren nach einem der Ansprüche 1 bis 3,
**dadurch gekennzeichnet,**
**dass** als Alkalimetallakoholat Natrium- oder Kaliummethanolat in Form einer Suspension oder Lösung in Methanol verwendet wird.

5. Verfahren nach einem der Ansprüche 1 bis 4,
**dadurch gekennzeichnet,**
**dass** als Malonsäureester der Dimethyl- oder der Diethylester verwendet wird.

6. Verfahren nach einem der Ansprüche 1 bis 5,
**dadurch gekennzeichnet,**
**dass** die Reaktion bei einem Druck von 1 bis 5 bar durchgeführt wird.

7. Verfahren nach einem der Ansprüche 1 bis 6,
**dadurch gekennzeichnet,**
**dass** das Reaktionsprodukt gewonnen wird, indem das Reaktionsgemisch mit Wasser versetzt und das Reaktionsprodukt durch Zusatz von Säure ausgefällt wird.

8. Verfahren nach einem der Ansprüche 1 bis 6,
**dadurch gekennzeichnet,**
**dass** aus dem alkoholischen Reaktionsgemisch der Alkohol in einem Dünnschichtversampfer abdestilliert wird, die zurückbleibenden Salze in Wasser gelöst werden und das 4,6-Dihydroxypyrimidin durch Zugabe von Säure ausgefällt wird.

## Revendications

1. Procédé de préparation de 4,6-dihydroxypyrimidine par réaction d'ester de l'acide malonique avec du formamide et un alcoolate de métal alcalin entre 30 et 100°C,
**caractérisé en ce qu'**
on ajoute l'ester de l'acide malonique seul ou en même temps que la totalité ou une partie du formamide, en plusieurs fois ou de façon continue, à l'alcoolate de métal alcalin disposé sous forme de solution ou de suspension dans un alcool, seul ou avec la totalité ou le reste du formamide, et on conduit la réaction sous la pression qui s'établit à la température de réaction correspondante.

2. Procédé selon la revendication 1,
**caractérisé en ce que**
la température dans le mélange réactionnel s'élève à 50 à 80°C tandis qu'on ajoute l'ester de l'acide malonique.

3. Procédé selon la revendication 1 ou 2,
**caractérisé en ce qu'**
on utilise par mole d'ester de l'acide malonique de 2,0 à 2,5, de préférence de 2,1 à 2,25 moles de formamide, et de 3,0 à 4,0, de préférence de 3,0 à 3,5 moles, d'alcoolate de métal alcalin.

4. Procédé selon l'une des revendications 1 à 3,
**caractérisé en ce qu'**
on utilise comme alcoolate de métal alcalin le méthanolate de sodium ou de potassium sous la forme d'une suspension ou d'une solution dans le méthanol.

5. Procédé selon l'une des revendications 1 à 4,
**caractérisé en ce qu'**
on utilise comme ester de l'acide malonique l'ester diméthylique ou l'ester diéthylique.

6. Procédé selon l'une dés revendications 1 à 5,
**caractérisé en ce qu'**
on conduit la réaction sous une pression comprise entre 1 et 5 bars.

7. Procédé selon l'une des revendications 1 à 6,
**caractérisé en ce qu'**
on obtient le produit de la réaction en mélangeant le mélange réactionnel avec de l'eau et en précipitant le produit de la réaction par addition d'acide.

8. Procédé selon l'une des revendications 1 à 6,
**caractérisé en ce qu'**
à partir du mélange réactionnel alcoolique on distille l'alcool dans un évaporateur en couche mince, on dissout dans l'eau les sels restants et on précipite la 4,6-dihydroxypyrimidine en ajoutant un acide.

## Claims

1. A process for the preparation of 4,6-dihydroxypyrimidine by reacting malonate with formamide and an alkali metal alkoxide at 30 to 100°C, **characterized in that** the malonate is added alone or simultaneously with all or some of the formamide in portions or continuously to the alkali metal alkoxide which has been initially introduced as a solution or suspension in an alcohol alone or together with all, or the remainder, of the formamide and **in that** the reaction is carried out under the pressure which is established at the reaction temperature in question.

2. A process according to claim 1, **characterized in that** the temperature in the reaction mixture is 50 to 80°C while the malonate is added.

3. A process according to either of claims 1 and 2, **characterized in that** 2.0 to 2.5, preferably 2.1 to 2.25, moles of formamide and 3.0 to 4.0, preferably 3.0 to 3.5, moles of alkali metal alkoxide are used per mole of malonate.

4. A process according to any one of claims 1 to 3, **characterized in that** sodium methoxide or potassium methoxide in the form of a suspension or solution in methanol is used as the alkali metal alkoxide.

5. A process according to any one of claims 1 to 4, **characterized in that** the dimethyl or the diethyl ester is used as the malonate.

6. A process according to any one of claims 1 to 5, **characterized in that** the reaction is carried out at a pressure of from 1 to 5 bar and **in that** the reaction is carried out under the pressure which is established at the reaction temperature in question.

7. A process according to any one of claims 1 to 6, **characterized in that** the reaction product is obtained by treating the reaction mixture with water and precipitating the reaction product by adding acid.

8. A process according to any one of claims 1 to 6, **characterized in that** the alcohol is removed from the alcoholic reaction mixture by distillation in a thin-film evaporator, the salt which remains is dissolved in water, and the 4,6-dihydroxypyrimidine is precipitated by adding acid.
